# EUROPEAN PATENT SPECIFICATION

(11) **EP 4 284 193 B1**
(45) Date of publication and mention of the grant of the patent: **07.05.2025**
(21) Application number: 22702425.4
(22) Date of filing: 21.01.2022
(51) Int. Cl.: A23L 33/135, A23L 33/21, A23L 33/00, A61K 31/702, A61K 31/745

(54) **MIXTURE OF HMOS AND BIFIDOBACTERIA**
MISCHUNG VON HMOS- UND BIFIDOBAKTERIEN
MÉLANGE DE HMO ET DE BIFIDOBACTÉRIES

(30) Priority: 29.01.2021 EP 21154300
(43) Date of publication of application: 06.12.2023
(60) Divisional application: 25167155.8
(73) Proprietor: Société des Produits Nestlé S.A., 1800 Vevey (CH)
(72) Inventor: DE BRUYN, Florac, 1006 LAUSANNE (CH); JOHNSON, Katja, 3510 KONOLFINGEN (CH); BOGICEVIC, Biljana, 3012 BERN (CH); MAES, Dominick, 1752 VILLARS-SUR-GLÂNE (CH); DUBOUX, Stéphane, 1162 St-Prex (CH)
(74) Representative: Gagliardi, Tatiana
(86) International application number: PCT/EP2022/051336
(87) International publication number: WO 2022/161865

(56) References cited:
- WO-A1-2017/103019
- WO-A1-2020/126587
- CN-A- 112 075 637
- US-A1- 2017 295 838

## Description

### FIELD OF THE INVENTION

The present invention relates to a composition for infants or young children comprising at least one probiotic bacterial strain, said probiotic bacterial strain belonging to bifidobacteria, and a prebiotic mixture of human milk oligosaccharides (HMO) consisting of 2'-fucosyllactose (2FL), difucosyllactose (DFL), 3'-sialyllactose (3SL), 6'-sialyllactose (6SL) and lacto-N-tetraose (LNT), and optionally 3-fucosyllactose (3FL) and applications of the mixtures in human health. The bifidobacteria comprises *Bifidobacterium longum* subsp. *infantis* or combination of *Bifidobacterium animalis* subsp. *lactis* and *Bifidobacterium longum* subsp. *infantis* thereof and at least one of the bifidobacteria is *Bifidobacterium longum* subsp. *infantis* LMG 11588 or a strain having an Average Nucleotide Identity (ANI) of at least 99.9% with this strain.

### BACKGROUND OF THE INVENTION

HMOs (human milk oligosaccharides) have become the subject of much interest in recent years due to their roles in numerous biological processes occurring in the human organism. Mammalian milk contains at least 130 of these complex oligosaccharides (Urashima et al, Milk Oligosaccharides, Nova Biomedical Books, New York, 2011, ISBN: 978-1-61122-831-1).

Evidence is accumulating that the resident community of microbes, called the microbiota, in the human digestive tract plays a major role in health and disease. When the composition of the intestinal microbiota is thrown off balance, the human host can suffer consequences. Recent research has implicated intestinal microbiota imbalances in individual disorders as diverse as cancer, obesity, inflammatory bowel disease, psoriasis, asthma, and possibly even autism. Individual non-digestible fibres, including HMOs, are believed to positively modulate the microbiota, and they are of increasing interest for treating one or more of such disorders.

Infancy, especially the first weeks, 3 months, 6 months or 12 months of life is a critical period for the establishment of a balanced gut microbiota.

It is known that the modulation of the gut microbiota during infancy can prospectively have a significant influence in the future health status of the body. For example, the gut microbiome can have an influence on the development of a strong immune system later in life, as well as normal growth, and even on the development of obesity later in life.

The gut microbiome and its evolution during the development of the infant is, however, a fine balance between the presence and prevalence (amount) of many populations of gut bacteria. Some gut bacteria are classified as "generally positive" while others are "generally negative" (or pathogenic) regarding their effect on the overall health of the infant. Certain species of "generally positive" bacteria, such as bifidobacteria, may be under-represented in infants fed conventional infant formula in comparison to breastfed infants. Similarly, some bacterial populations are considered pathogenic and should remain at a low prevalence in the gut microbiota.

*Bifidobacterium longum* subsp. *infantis* has been demonstrated to predominate in the gut microbiota of breastfed infants and to benefit the host by accelerating maturation of the immune response, balancing the immune system to suppress inflammation, improving intestinal barrier function, and increasing short-chain fatty acid production. Reduced abundance of *Bifidobacterium* species in infants has been correlated to chronic diseases, including asthma and obesity, as well as to lower vaccine response. Researchers have postulated that loss of *Bifidobacterium* species in the infant gut in populations of developed countries is linked to increased incidence of allergic and autoimmune diseases.

Increasing the abundance of *Bifidobacterium* species or specifically *Bifidobacterium longum* subsp. *infantis* in the gut microbial ecosystem through exogenous administration may be difficult to achieve, especially in formula-fed infants.

Key metabolites produced by *Bifidobacterium longum* subsp. *infantis* are lactic acid and acetic acid. Lactic and acetic acid contribute to decreasing stool pH and providing colonization resistance against pathogens in infants (Duar RM, Kyle D and Casaburi G, Colonization Resistance in the Infant Gut: The Role of B. infantis in Reducing pH and Preventing Pathogen Growth; High-Throughput, 2020). Both acids can serve as substrates for other members of the microbiota that produce other short-chain fatty acids (SCFA). SCFA are especially produced by microbial fermentation of dietary fibers in the colon. These colonic fermentations have been known to play a role in energy supply, as trophic factors, and in immune regulation and growing evidence suggests that SCFAs also exert important physiological effects on several organs, including the brain. High abundance of bacteria producing SCFA, particularly butyrate, has been shown to be linked to milder atopic eczema in infants (Nylund L et al., Severity of Atopic disease inversely correlates with intestinal microbiota diversity and butyrate-producing bacteria, Allergy, 2015).

CN 112075637 A relates to the use of a nutritional composition for: (a) reducing production of intestinal gas in infants; and/or (b) inhibiting excessive growth of *Clostridium aerogenes* in intestinal tracts of infants, wherein the nutritional composition comprises: (i) a breast milk oligosaccharide (HMO); and (ii) a probiotic microorganism, wherein the probiotic microorganism comprises bifidobacteria.

US 2017/295838 A1 relates to an age-tailored nutritional composition system comprising at least two nutritional compositions for infants of a certain age, which differ from each other in the amount of HMOs present therein, and a method for preparing said system.

WO 2020/126587 A1 relates to nutritional compositions comprising metabolites of HMOs to improve the gastrointestinal barrier.

WO 2017/103019 A1 relates to a mixture of human milk oligosaccharides and composition comprising thereof, that consists essentially of: LNnT, LNT, 2'-FL, 3'-SL, 6'-SL and either DFL or 3-FL, preferably DFL, that can be useful for preventing and/or treating viral and/or bacterial infections in a human.
Due to the loss of *Bifidobacterium* species in the infant gut and low breast-feeding rates, there is a need to provide infants with both, HMOs and HMO-utilizing bacteria such as *B. longum* subsp. *infantis* to support a healthy microbiome for long-term health.

### SUMMARY OF THE INVENTION

A first aspect of this invention relates to a composition for infants or young children comprising at least one probiotic bacterial strain, said probiotic bacterial strain being bifidobacteria, and a prebiotic oligosaccharides mixture consisting of 2'-fucosyllactose (2FL), lactodifucotetraose/difucosyllactose (DFL), lacto-N-tetraose (LNT), 6'-sialyllactose (6SL), and 3'-sialyllactose (3SL) and optionally 3-fucosyllactose (3FL);
wherein the bifidobacteria comprises *Bifidobacterium longum* subsp. *infantis* or combination of *Bifidobacterium animalis* subsp. *lactis* and *Bifidobacterium longum* subsp. *infantis* thereof;
wherein at least one of the bifidobacteria is *Bifidobacterium longum* subsp. *infantis* LMG 11588 or a strain having an Average Nucleotide Identity (ANI) of at least 99.9% with this strain

The present inventors have surprisingly found that a synergistic effect is obtained when this HMO mix is used in combination with *Bifidobacterium,* in particular the combination of the probiotic, for instance *B. longum* subsp. *infantis,* with the HMO mix synergistically promotes a healthier gut environment due to the enhancement of beneficial fermentative metabolites such as short chain fatty acids and the growth stimulation of healthy commensal gut bacteria such as bifidobacteria.

In one aspect the present invention relates to a composition wherein the bifodobacteria is *Bifidobacterium longum* subsp. *infantis* and the prebiotic oligosaccharides mixture consists of 2'-fucosyllactose (2FL), lactodifucotetraose/difucosyllactose (DFL), lacto-N-tetraose (LNT), 6'-sialyllactose (6SL), and 3'-sialyllactose (3SL).

In another aspect the present invention relates to a composition wherein the bifodobacteria is a combination of *Bifidobacterium animalis* subsp. *lactis* and *Bifidobacterium longum* subsp. *infantis* and the prebiotic oligosaccharides mixture consists of 2'-fucosyllactose (2FL), lactodifucotetraose/difucosyllactose (DFL), lacto-N-tetraose (LNT), 6'-sialyllactose (6SL), and 3'-sialyllactose (3SL).

In one aspect the present invention relates to a nutritional composition selected from the list consisting of an infant formula, a starter infant formula, a follow-on or follow-up formula, a baby food, an infant cereal composition, growing-up-milk, a fortifier such as a human milk fortifier, or a supplement.

In one aspect the present invention relates to a nutrional composition as defined above for use in: i) preventing and/or treating bacterial infections in an infant or a young child; ii) modulating the microbiota of an infant or a young child; and/or iii) preventing and/or treating allergy of an infant or a young child.

In one aspect the present invention relates to a composition as defined above for use for modulating the microbiota of an infant or a young child and administration of said composition results in abundance of Bifidobacteriaceae and/or *Bifidobacterium longum* subsp. *infantis* being increased.

In one aspect the present invention relates to a composition as defined above for modulating the microbiota of an infant or a young child; and/or preventing and/or treating allergy of an infant or a young child by increasing intestinal short-chain fatty acids (SCFA) production in such infant or young child.

Described herein is a method of modulating the microbiota of an infant or a young child to increase the abundance of Bifidobacteriacea and/or *Bifidobacterium longum* subsp. *infantis,* the method comprising:
- administering, to the infant or young child, a composition as defined herein.

### FIGURES

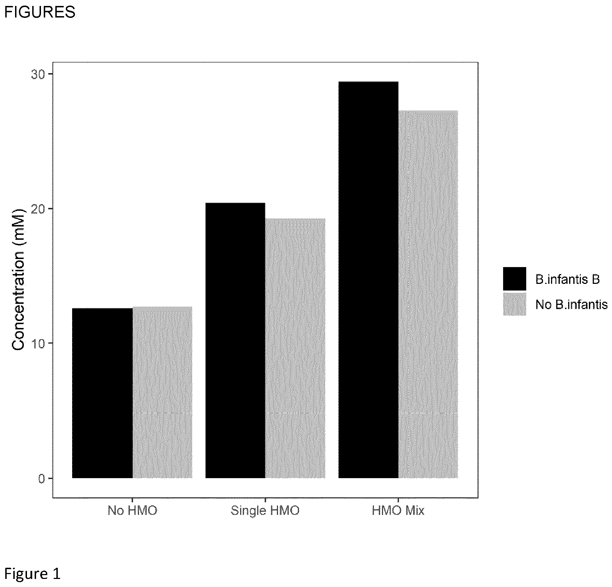

**Figure 1****:** Total short chain fatty acid concentration after 24 h averaged over 8 faecal donors. Average concentrations increase upon supplementation of a single HMO (2FL). A larger increase occurs when supplementing an HMO mix instead of a single HMO. Further incremental increases are obtained when supplementing *Bifidobacterium infantis B. Bifidobacterium infantis* B is B. *longum* subsp. *infantis* LMG 11588.

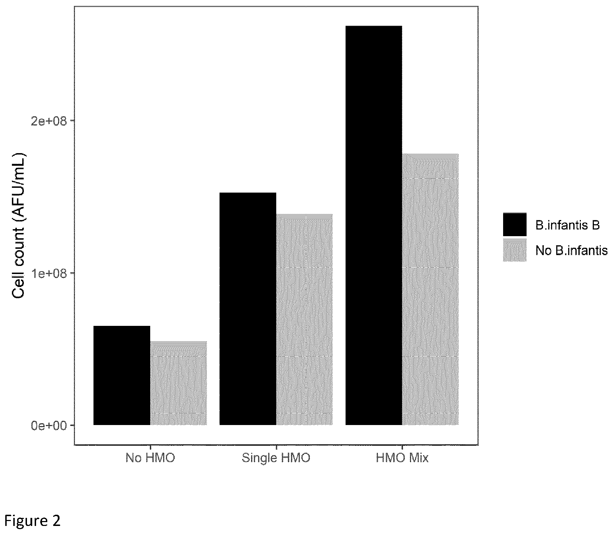

**Figure 2****:** Prevalence of the Bifidobacteriaceae operational taxonomic unit after 24 h averaged over 8 faecal donors. Average prevalence increases upon supplementation of a single HMO (2FL). A larger increase occurs when supplementing an HMO mix instead of a single HMO. Further incremental increases are obtained when supplementing *Bifidobacterium infantis B. Bifidobacterium infantis* B is B. *longum* subsp. *infantis* LMG 11588.

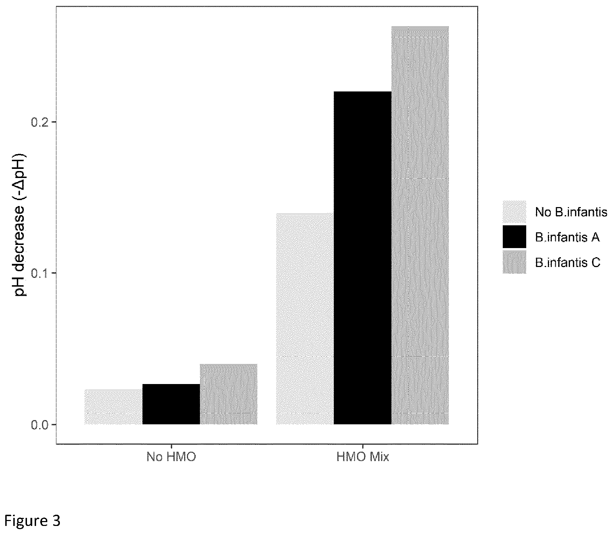

**Figure 3****:** Total acidification after 48h (average of triplicates). Average concentrations increase upon supplementation of an HMO mix. Sequential increases are obtained when supplementing respectively *Bifidobacterium infantis* A and *Bifidobacterium infantis* C in the presence of an HMO mix. *Bifidobacterium infantis* A is B. *longum* subsp. *infantis* ATCC 15697. *Bifidobacterium infantis* C is a strain with more than 99.9% ANI with B. *longum* subsp. *infantis* LMG 11588.

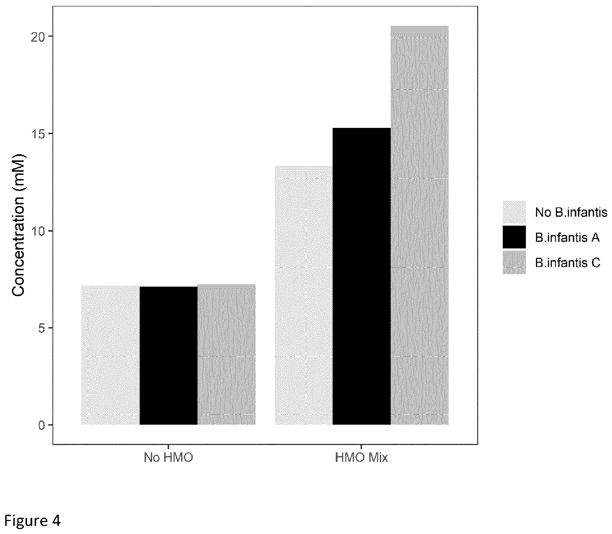

**Figure 4****:** Total short chain fatty acid concentration after 48h (average of triplicates). Average concentrations increase upon supplementation of an HMO mix. Sequential increases are obtained when supplementing respectively *Bifidobacterium infantis* A and *Bifidobacterium infantis* C in the presence of an HMO mix. *Bifidobacterium infantis* A is *B. longum* subsp. *infantis* ATCC 15697. *Bifidobacterium infantis* C is a strain with more than 99.9% ANI with B. *longum* subsp. *infantis* LMG 11588.

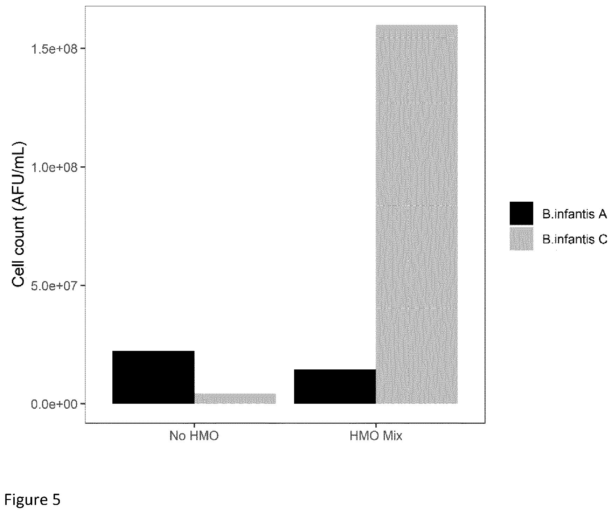

**Figure 5****:** Prevalence of the *Bifidobacterium* infantis-related operational taxonomic unit after 48h (average of triplicates). Largest increase is obtained when supplementing *Bifidobacterium infantis* C in the presence of an HMO mix. *Bifidobacterium infantis* A is *B. longum* subsp. *infantis* ATCC 15697. *Bifidobacterium infantis* C is a strain with more than 99.9% ANI with B. *longum* subsp. *infantis* LMG 11588.

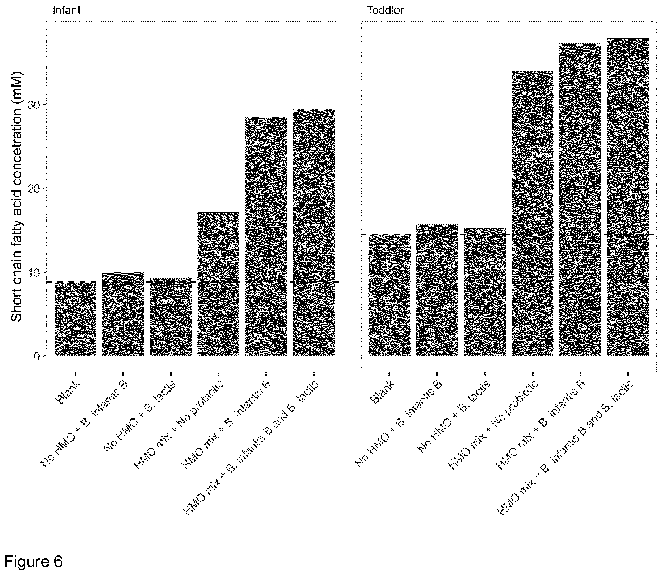

**Figure 6****:** Total short chain fatty acid concentration after 24 h averaged over 6 faecal donors at infant age (3 months old) and toddler age (12 months old). There is no notable increase in average total short chain fatty acid concentrations compared to the blank when supplementing *Bifidobacterium infantis* B without HMOs or *Bifidobacterium lactis* without HMOs. Average concentrations increase upon supplementation of an HMO mix alone. Further increases are obtained when supplementing *B. infantis* B with an HMO mix and with the combination of B. *infantis* B and *B*. *lactis* with an HMO mix. A horizontal dashed line is added to denote the blank level of total short chain fatty acid concentration for infants and toddlers. *Bifidobacterium infantis* B is *Bifidobacterium longum* subsp. *infantis* LMG 11588. *Bifidobacterium lactis* is *Bificobacterium animalis* subsp. *lactis* CNCM I-3446.

### DETAILED DESCRIPTION OF THE INVENTION

As used herein, the following terms have the following meanings.

The term "**infant**" means a child under the age of 12 months.

The expression **"young child"** means a child aged between one and three years, also called toddler.

The expressions **"a composition for infants or young children"** and **"a composition to be administered to infants or young children"** can be used interchangeably.

In some embodiments, the composition comprising the HMOs mixture according to the invention is a nutritional composition. The expression **"nutritional composition"** means a composition which nourishes a subject. This nutritional composition is usually to be taken orally or intravenously, and it usually includes a lipid or fat source and a protein source. In a particular embodiment, the nutritional composition is a synthetic nutritional composition.

The expression **"infant formula"** as used herein refers to a foodstuff intended for particular nutritional use by infants during the first months of life and satisfying by itself the nutritional requirements of this category of person (Article 2(c) of the European Commission Directive 91/321/EEC 2006/141/EC of 22 December 2006 on infant formulae and follow-on formulae). It also refers to a nutritional composition intended for infants and as defined in Codex Alimentarius (Codex STAN 72-1981) and Infant Specialities (incl. Food for Special Medical Purpose). The expression "infant formula" encompasses both "starter infant formula" and "follow-up formula" or "follow-on formula".

A **"follow-up formula"** or **"follow-on formula"** is given from the 6th month onwards. It constitutes the principal liquid element in the progressively diversified diet of this category of person.

The expression **"baby food"** means a foodstuff intended for particular nutritional use by infants or young children during the first years of life.

The expression **"infant cereal composition"** means a foodstuff intended for particular nutritional use by infants or young children during the first years of life.

The term **"fortifier"** refers to liquid or solid nutritional compositions suitable for mixing with breast milk or infant formula. In one embodiment, the aqueous composition according to the present invention is a milk fortifier. In such embodiment, the aqueous composition of the invention may be packed in single doses.

The term **"supplement"** means a foodstuff containing specific nutrients and/or probiotics intended to supplement a diet.

In one embodiment, the present invention is a supplement in form of powder or an oil. In such embodiment, the composition of the invention is administered as a standalone composition, or combined with other ingredients such as maltodextrin.

The term **"growing-up milk"** (or GUM) refers to a milk-based drink generally with added vitamins and minerals, that is intended for young children or children.

**HMO** (Human Milk Oligosaccharides) are Oligosaccharide structures that naturally occur in human milk. The HMOs that are added to foodstuffs are typically obtained from cow milk, by chemical synthesis or by a biotechnological production process.

The term **"SCFA"** means short chain fatty acid(s).

The expression **"increasing SCFA production"** means that the amount of systemic and/or colonic SCFA, is higher in an individual fed with the nutritional composition according to the present invention in comparison with a standard. The SCFA production may be measured by techniques known by the skilled person such as by Gas-Liquid Chromatography.

The **"Average Nucleotide Identity (ANI)"** is a measure of nucleotide-level genomic similarity between the coding regions of two genomes. Average Nucleotide Identity can be assessed as describe here: Yoon SH, Ha SM, Lim J, Kwon S, Chun J. A large-scale evaluation of algorithms to calculate average nucleotide identity. Antonie Van Leeuwenhoek. 2017 Oct;110(10):1281-1286. In the present embodiment the strain *Bifidobacterium longum* subsp. *infantis* LMG 11588 (also known as ATCC 17930) represents the reference genome to which a microbial genome is compared. An example of a microorganism genome that has at least 99.9% ANI with *B*. *longum* subsp. *infantis* LMG 11588 can be found in PATRIC (https://www.patricbrc.org), genome ID 1678.111 (strain C). In one embodiment of the present invention, the *Bifidobacterium longum* subsp. *infantis* strain does not harbour potentially transferable antibiotic resistances.

The HMO and bifidobacteria mixture of this invention can:
i) Increase the indigenous intestinal abundance of Bifidobacteriacea and
ii) Increase intestinal SCFA production and prolong faecal pH decrease and
iii) Support growth of *B*. *longum* subsp. *infantis*

The effect on bifidobacteria and SCFA of the HMO and bifidobacteria mixture is greater than expected from the effects of bifidobacteria or HMO mix alone. The HMO and bifidobacteria mixture of this invention has thus a surprising synergistic effect.

These effects can render an intestinal milieu less prone to invasion and overgrowth of harmful bacteria in the infant gut. These effects of the HMO and bifidobacteria mixture may prevent and/or treat conditions such as allergies, bacterial infections, inflammatory bowel disease, irritable bowel syndrome, obesity and other conditions associated with inflammation and impaired barrier function.

In one embodiment, the bifidobacteria further comprises *Bifidobacterium animalis* subsp. *lactis CNCM 1-3446.*

In one embodiment, the HMO mixture of this invention consists essentially of:
i. 34 wt% to 85 wt% of 2FL, preferably 42wt% to 71 wt%;
ii. 10 wt% to 40 wt% of LNT, preferably 14 wt% to 26 wt%;
iii. 4 wt% to 14 wt% of DFL, preferably 5 wt% to 10 wt%; and
iv. 9 wt% to 31 wt% of 6SL and 3SL combined, preferably 10 wt% to 28 wt%.

In another embodiment, the HMO mixture of this invention consists essentially of:
i. 26 wt% to 65 wt% of 2FL, preferably 32 wt% to 54 wt%;
ii. 8 wt% to 30 wt% of LNT, preferably 11 wt% to 20 wt%;
iii. 3 wt% to 11 wt % of DFL, preferably 4 wt% to 8 wt%;
iv. 7wt% to 23wt % of 6SL and 3SL combined, preferably 8wt % to 22wt %; and
v. 12wt% to 38wt% of 3FL, preferably 17wt% to 31wt%.

In one embodiment, the HMO mixture of this invention consists essentially of:
i. 40 wt% to 80 wt% of 2FL, preferably 50wt% to 70 wt%;
ii. 10 wt% to 40 wt% of LNT, preferably 14 wt% to 26 wt%;
iii. 4 wt% to 14 wt% of DFL, preferably 5 wt% to 10 wt%; and
iv. 9 wt% to 31 wt% of 6SL and 3SL combined, preferably 10 wt% to 28 wt%.

In another embodiment, the HMO mixture of this invention consists essentially of:
i. 20 wt% to 60 wt% of 2FL, preferably 25 wt% to 55 wt%;
ii. 8 wt% to 30 wt% of LNT, preferably 11 wt% to 20 wt%;
iii. 2 wt% to 12 wt % of DFL, preferably 4 wt% to 8 wt%;
iv. 7wt% to 23wt % of 6SL and 3SL combined, preferably 8wt % to 22wt %; and
v. 10wt% to 40wt% of 3FL, preferably 11wt% to 37wt%.

The HMO and bifidobacteria mixture can be administered to a human in any suitable form such as, for example, a nutritional composition in a unit dosage form (for example, a tablet, a capsule, a sachet of powder, etc.).

The HMO and bifidobacteria mixture of this invention can also be added to a nutritional composition. For example, it can be added to an infant formula, a food composition, a rehydration solution, or a dietary maintenance or supplement for infants or young children. The nutritional composition can be for example an infant formula, a starter infant formula, a follow-on or follow-up formula, a baby food, an infant cereal composition, a fortifier such as a human milk fortifier, or a supplement. In some particular embodiments, the composition of the invention is an infant formula, a fortifier or a supplement that may be intended for the first 4 or 6 months of age. In a preferred embodiment the nutritional composition of the invention is an infant formula. In some other embodiments the nutritional composition of the present invention is a fortifier. The fortifier can be a breast milk fortifier (e.g. a human milk fortifier) or a formula fortifier such as an infant formula fortifier or a follow-on/follow-up formula fortifier.

Macronutrients such as edible fats, carbohydrates and proteins can also be included to such a nutritional composition. Edible fats include, for example, coconut oil, soy oil and monoglycerides and diglycerides. Carbohydrates include, for example, glucose, edible lactose and hydrolysed corn starch. Proteins include, for example, soy protein, whey, and skim milk. Vitamins and minerals (e. g. calcium, phosphorus, potassium, sodium, chloride, magnesium, manganese, iron, copper, zinc, selenium, iodine, and Vitamins A, **E,** D, C, and B complex) can also be included in such a nutritional composition.

The nutritional composition may be prepared in any suitable manner. A composition will now be described by way of example.

For example, a formula such as an infant formula may be prepared by blending together the protein source, the carbohydrate source and the fat source in appropriate proportions. If used, the emulsifiers may be included at this point. The vitamins and minerals may be added at this point but they are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently in the range between about 50°C and about 80°C to aid dispersal of the ingredients. Commercially available liquefiers may be used to form the liquid mixture.

The HMO mixture of the present invention may be added at this stage, especially if the final product is to have a liquid form. If the final product is to be a powder, they may likewise be added at this stage if desired.

The liquid mixture is then homogenised, for example in two stages.

The liquid mixture may then be thermally treated to reduce bacterial loads, by rapidly heating the liquid mixture to a temperature in the range between about 80°C and about 150°C for a duration between about 5 seconds and about 5 minutes, for example. This may be carried out by means of steam injection, an autoclave or a heat exchanger, for example a plate heat exchanger.

Then, the liquid mixture may be cooled to between about 60°C and about 85°C for example by flash cooling. The liquid mixture may then be again homogenised, for example in two stages between about 10 MPa and about 30 MPa in the first stage and between about 2 MPa and about 10 MPa in the second stage. The homogenised mixture may then be further cooled to add any heat sensitive components, such as vitamins and minerals. The pH and solids content of the homogenised mixture are conveniently adjusted at this point.

If the final product is to be a powder, the homogenised mixture is transferred to a suitable drying apparatus such as a spray dryer or freeze dryer and converted to powder. The powder should have a moisture content of less than about 5% by weight. The HMO mixture of the present invention may also or alternatively be added at this stage by dry-mixing along with the probiotic strain(s) in powder.

Preferred features and embodiments of the invention will now be described by way of nonlimiting examples.

The practice of the present invention will employ, unless otherwise indicated, conventional techniques of chemistry, biochemistry, molecular biology, microbiology and immunology, which are within the capabilities of a person of ordinary skill in the art. Such techniques are explained in the literature. See, for example, Sambrook, J., Fritsch, E.F. and Maniatis, T. (1989) Molecular Cloning: A Laboratory Manual, 2nd Edition, Cold Spring Harbor Laboratory Press; Ausubel, F.M. et al. (1995 and periodic supplements) Current Protocols in Molecular Biology, Ch. 9, 13 and 16, John Wiley & Sons; Roe, B., Crabtree, J. and Kahn, A. (1996) DNA Isolation and Sequencing: Essential Techniques, John Wiley & Sons; Polak, J.M. and McGee, J.O'D. (1990) In Situ Hybridization: Principles and Practice, Oxford University Press; Gait, M.J. (1984) Oligonucleotide Synthesis: A Practical Approach, IRL Press; and Lilley, D.M. and Dahlberg, J.E. (1992) Methods in Enzymology: DNA Structures Part A: Synthesis and Physical Analysis of DNA, Academic Press.

### Examples

### Example 1 - Investigation of synbiotic effect of B. longum subsp. infantis LMG 11588 in combination with 2FL or 5 HMO mix

### Material & Methods

Fecal material was collected from 10 approximately 3-month-old donors in Belgium. 4 donors were born by C-section and 6 were vaginally born. One donor was breast-fed, the other 9 donors were infant formula fed in the week before sampling. Fecal suspensions were prepared, mixed with cryoprotectant, aliquoted, flash frozen and then preserved at -80°C. Just before the experiment, fecal samples were defrosted and immediately used in the experiments.

The fecal material was used to perform short-term batch fermentation experiments that mimic the infant colon. These experiments represent a simplified simulation of the continuous Simulator of the Human Microbial Ecosystem (SHIME^{®}). At the start of the short-term colonic incubations, test ingredients were added to a sugar-depleted nutritional medium containing basal nutrients of the colon. Because nutrients of this sugar-depleted nutritional medium will also be fermented by the colonic microbiota, a blank containing only the sugar-depleted nutritional medium (without product) was included for each donor. Finally, fecal inocula of the infant donors were added. Five treatments and one blank were tested per donor, resulting in 60 independent experiments. Treatments were as follows:
1. Blank (control)
2. Single HMO (1.3 g/L)
3. 5 HMO mix (2.5 g/L)
*4. B. longum* subsp. *infantis* LMG 11588 (1×10⁷ CFU/ml)
5. Single HMO (1.3 g/L) + *B. longum* subsp. *infantis* LMG 11588 (1×10⁷ CFU/ml)
6. 5 HMO mix (2.5 g/L) + *B. longum* subsp. *infantis* LMG 11588 (1×10⁷ CFU/ml)

The single HMO was 2'-fucosyllactose (2FL). The HMO mix consisted of 5 HMOs in the following ratio in the dry mix: 23% LNT, 9% 6SL, 2% 3SL, 52% 2FL & 14% DFL. Therefore the addition rate of 2FL was the same in the single HMO and HMO mix treatment.

Reactors were incubated for 48h at 37°C, under shaking and anaerobic conditions. The incubations were performed in fully independent reactors with sufficiently high volume to not only ensure robust microbial fermentation, but also to allow the collection of multiple samples over time. Sample collection enables assessment of metabolite production and thus to understand the complex microbial interactions that are taking place.

An assessment was made amongst others on short chain fatty acids (SCFA) production at the start of the incubation and after 6h, 24h and 48h. The pattern of SCFA production is an assessment of the microbial carbohydrate metabolism.

Changes in microbial composition were analysed at the start and after 24h and 48h of incubation. Samples were analyzed with a combination of 16S-targeted Illumina sequencing and flow cytometry to determine the number of bacterial cells of each bacterial community present, thus allowing to convert the proportional values obtained with Illumina into absolute cell counts.

Two donors (both vaginally born and infant formula fed) were excluded from the result evaluation as the sequencing results indicated technical or analytical problems.

### Results

Figure 1 shows the short chain fatty acid (SCFA) concentration obtained in the colonic fermentations averaged over 8 faecal donors. When only B. *longum* subsp. *infantis* LMG 11588 was added, no increase in SCFA occurred compared to the control (no HMO, no B. *infantis).* With the single HMO 2FL, an increase versus the control could be observed; this increase was even greater with the HMO mix. However, when B. *longum* subsp. *infantis* was added on top of the single HMO or HMO mix, an additional increase in SCFA concentration was achieved compared to not adding *B*. *longum* subsp. *infantis.* The effect of the combinations of the single HMO or HMO mix with B. *longum* subsp. *infantis* versus the control was greater than adding up the effects of the individual ingredients versus the control, thus showing a clear synergistic (synbiotic) effect. The greatest increase in SCFA can be obtained with the combination of the HMO mix and B. *longum* subsp. *infantis.*

Figure 2 shows the cell count of the Bifidobacteriaceae family obtained in the colonic fermentations averaged over 8 faecal donors. *Bifidobacterium longum* subsp. *infantis* LMG 11588 was added at a level of 1×10⁷ CFU/ml in the variants where it was added. When only *B. longum* subsp. *infantis* was added, a small increase in Bifidobacteriaceae compared to the control (no HMO, no *B*. *infantis*) was observed. With the single HMO 2FL, an increase in Bifidobacteriaceae versus the control could be seen; this increase was even greater with the HMO mix. However, when *B*. *longum* subsp. *infantis* was added on top of the the single HMO or HMO mix, an additional increase in Bifidobacteriaceae was achieved compared to not adding *B. longum* subsp. *infantis.* For the combination of the HMO mix and *B. longum* subsp. *infantis,* the increase in Bifidobacteriaceae versus the control was greater than the individual effects of either ingredient versus the control, thus showing a clear synergistic (synbiotic) effect. Thus either *B. longum* subsp. *infantis* LMG 11588 has grown and/or its addition has stimulated growth of other members of the Bifidobacteriaceae family. The combination of the HMO mix and *B. longum* subsp. *infantis* was thus most effective in increasing the number of Bifidobacteriaceae. The overall results correlate with the SCFA data shown above.

### Example 2 - Investigation of synbiotic effect of 2 different strains of B. longum subsp. infantis in combination with 5 HMO mix

### Material & Methods

Fecal material was collected from a 3-month-old baby donor in Belgium. The donor was different from the 10 donors in Example 1. The experiment was similar to Example 1, but all colonic fermentations were carried out in triplicate and the test ingredients differed. Five treatments and one blank were tested, resulting in 18 experiments in total. Treatments were as follows:
1. Blank (Control)
2. 5 HMO mix (2.5g/L)
3. *B. longum* subsp. *infantis A* (1E+07 cfu/ml)
4. *B. longum* subsp. *infantis C* (1E+07 cfu/ml)
5. *B. longum* subsp. *infantis A* (1E+07 cfu/ml) + 5 HMO mix (2.5g/L)
6. *B. longum* subsp. *infantis* C (1E+07 cfu/ml) + 5 HMO mix (2.5g/L)

*B. longum* subsp. *infantis* A is the strain ATCC 15697, which is the type strain of the subspecies and shares less than 99.9% ANI (98.2%) with *B*. *longum* subsp. *infantis* LMG 11588. The strain *B. longum* subsp. *infantis* C has more than 99.9% ANI with *B. longum* subsp. *infantis* LMG 11588 and is thus closely related with the LMG 11588 strain.

The HMO mix consisted of 5 HMOs in the following ratio in the dry mix: 23% LNT, 9% 6SL, 2% 3SL, 52% 2FL & 14% DFL.

An assessment was made amongst others on pH and short chain fatty acids (SCFA) production at the start of the incubation and after 6h, 24h and 48h.

Changes in microbial composition were analysed at the start and after 24h and 48h of incubation. Samples were analyzed with a combination of 16S-targeted Illumina sequencing and flow cytometry to determine the number of bacterial cells of each bacterial community present, thus allowing to convert the proportional values obtained with Illumina into absolute cell counts.

### Results

Figures 3 and 4 show that the addition of either *B. longum* subsp. *infantis* strain alone did not have an impact on acidification or SCFA production in this donor. The addition of the HMO mix alone resulted in a marked increase in SCFAs. Production of SCFAs could be further increased with the combination of the HMO mix with either *B*. *longum* subsp. *infantis* strain. The best effect was achieved with the combination of the HMO mix with *B. longum* subsp. *infantis* C - the strain that has more than 99.9% ANI with *B. longum* subsp. *infantis* LMG 11588. The effects of the HMO mix and *B*. *longum* subsp. *infantis* combinations versus the control (no HMO, no *B. infantis)* was greater than the sum of the effects of single ingredients versus the control, thus showing a clear synbiotic effect. This synbiotic effect was more pronounced with *B. longum* subsp. *infantis* C than with *B. longum* subsp. *infantis A.*

The sequencing data revealed that the infant donor had no detectable *B. longum* subsp. infantis-related operational taxonomic unit present in the inoculum, but it could be detected after addition of the two strains. After 48h colonic simulation (Figure 5) levels of *B. longum* subsp. *infantis* remained low for both strains when no HMO were added. However, for *B. longum* subsp. *infantis* C in combination with the HMO mix a large increase in B. *longum* subsp. infantis-related operational taxonomic was detected, indicating a strong growth stimulation of the species. This shows again the synergistic effect that can be obtained with the combination of the HMO mix with *B. longum* subsp. *infantis.*

### Example 3 - Investigation of synbiotic effect of B. longum subsp. infantis LMG 11588 in combination with 6 HMO mix with and without B. lactis in an infant and toddler colonic model

### Material & Methods

Fecal material was collected from 6 donors in Belgium at about 3 months of age (infants) and from same donors at about 12 months of age (toddlers). 3 donors were born by C-section and 3 were vaginally born. One donor was breast-fed, the other 5 donors were infant formula fed in the week before the first sampling. The experiment was similar to Example 1. Five treatments and one blank were tested per donor at two ages, resulting in 72 independent experiments. Treatments were as follows:
1. Blank (control)
2. *B. longum* subsp. *infantis* LMG 11588 (1×10⁸ CFU/ml)
3. *B. animalis* subsp. *lactis* CNCM I-3446 (1×10⁸ CFU/ml)
4.6 HMO mix (2.5 g/L)
5.6 HMO mix (2.5 g/L) + *B. longum* subsp. *infantis* LMG 11588 (1×10⁸ CFU/ml)
6. 6 HMO mix (2.5 g/L) + *B. longum* subsp. *infantis* LMG 11588 (1×10⁸ CFU/ml) + *B. animalis* subsp. *lactis* CNCM I-3446 (1×10⁸ CFU/ml)

The HMO mix consisted of 6 HMOs in the following ratio in the dry mix: for the infant study 16% LNT, 8% 6SL, 6% 3SL, 49% 2FL, 7% DFL & 14% 3FL; for the toddler study 10% LNT, 5% 6SL, 14% 3SL, 29% 2FL, 4% DFL & 37% 3FL.

An assessment was made amongst others on short chain fatty acids (SCFA) production at the start of the incubation and after 6h, 24h and 48h.

### Results

Figure 6 shows the short chain fatty acid (SCFA) concentration obtained in the colonic fermentations at 24h averaged over 6 faecal donors at the two different ages. When only *B. longum* subsp. *infantis* LMG 11588 or *B. animalis* subsp. *lactis* CNCM I-3446 were added, no meaningful increase in SCFA occurred compared to the blank control for both models, infants and toddlers. With the 6 HMO mix, an increase in SCFA concentration versus the blank control could be observed; the effect was stronger in the the toddler model than in the infant model. However, when *B. longum* subsp. *infantis* was added additionally to the 6 HMO mix, an additional increase in SCFA concentration was achieved compared to not adding *B. longum* subsp. *infantis.* The additional effect of *B. longum* subsp. *infantis* addition was stronger in the infant model than in the toddler model. The effect of the combination of the HMO mix with *B. longum* subsp. *infantis* versus the control was greater than adding up the effects of the individual ingredients versus the blank control in both models, thus showing a clear synergistic (synbiotic) effect. The synbiotic effect of the HMO mix with B. *longum* subsp. *infantis* was maintained when additionally *B. animalis* subsp. *lactis* was added; addition of *B. animalis* subsp. *lactis* led to a slight further increase in SCFA concentration. The greatest increase in SCFA can be obtained with the combination of the 6 HMO mix with *B. longum* subsp. *infantis* and with *B. animalis* subsp. *lactis* in both, infant and toddler model.

## Claims

1. A composition for infants or young children comprising at least one probiotic bacterial strain, said probiotic bacterial strain being bifidobacteria, and a prebiotic oligosaccharides mixture consisting of 2'-fucosyllactose (2FL), lactodifucotetraose/difucosyllactose (DFL), lacto-N-tetraose (LNT), 6'-sialyllactose (6SL), and 3'-sialyllactose (3SL) and optionally 3-fucosyllactose (3FL);
wherein the bifidobacteria comprises *Bifidobacterium longum* subsp. *infantis* or combination of *Bifidobacterium animalis* subsp. *lactis* and *Bifidobacterium longum* subsp. *infantis;*
wherein at least one of the bifidobacteria is *Bifidobacterium longum* subsp. *infantis* LMG 11588 or a strain having an Average Nucleotide Identity (ANI) of at least 99.9% with this strain.

2. The composition of claim 1, wherein the bifidobacteria comprises *Bifidobacterium longum* subsp. *infantis* and the prebiotic oligosaccharides mixture consists of 2'-fucosyllactose (2FL), lactodifucotetraose/difucosyllactose (DFL), lacto-N-tetraose (LNT), 6'-sialyllactose (6SL), and 3'-sialyllactose (3SL).

3. The composition of claim 1, wherein the bifidobacteria is a combination of *Bifidobacterium animalis* subsp. *lactis* and *Bifidobacterium longum* subsp. *infantis* and the prebiotic oligosaccharides mixture consists of 2'-fucosyllactose (2FL), lactodifucotetraose/difucosyllactose (DFL), lacto-N-tetraose (LNT), 6'-sialyllactose (6SL), and 3'-sialyllactose (3SL).

4. The composition of any one of the claims 1 to 3, wherein at least one of the bifidobacteria is *Bifidobacterium animalis* subsp. *lactis* CNCM I-3446.

5. The composition according to any one of the preceding claims, wherein the mixture of HMOs consists essentially of:
i. 34 wt% to 85 wt% of 2FL, preferably 42wt% to 71 wt%;
ii. 10 wt% to 40 wt% of LNT, preferably 14 wt% to 26 wt%;
iii. 4 wt% to 14 wt% of DFL, preferably 5 wt% to 10 wt%; and
iv. 9 wt% to 31 wt% of 6SL and 3SL combined, preferably 10 wt% to 28 wt%.

6. The composition according to any one of the claims 1 to 4 wherein the mixture of HMOs consists essentially of:
i. 26 wt% to 65 wt% of 2FL, preferably 32 wt% to 54 wt%;
ii. 8 wt% to 30 wt% of LNT, preferably 11 wt% to 20 wt%;
iii. 3 wt% to 11 wt % of DFL, preferably 4 wt% to 8 wt%;
iv. 7wt% to 23wt % of 6SL and 3SL combined, preferably 8wt % to 22wt %; and
v. 12wt% to 38wt% of 3FL, preferably 17wt% to 31wt%

7. The composition according to any one of the claims 1 to 4, wherein the mixture of HMOs consists essentially of:
i. 40 wt% to 80 wt% of 2FL, preferably 50wt% to 70 wt%;
ii. 10 wt% to 40 wt% of LNT, preferably 14 wt% to 26 wt%;
iii. 4 wt% to 14 wt% of DFL, preferably 5 wt% to 10 wt%; and
iv. 9 wt% to 31 wt% of 6SL and 3SL combined, preferably 10 wt% to 28 wt%.

8. The composition according to any one of the claims 1 to 4, wherein the mixture of HMOs consists essentially of:
i. 20 wt% to 60 wt% of 2FL, preferably 25 wt% to 55 wt%;
ii. 8 wt% to 30 wt% of LNT, preferably 11 wt% to 20 wt%;
iii. 2 wt% to 12 wt % of DFL, preferably 4 wt% to 8 wt%;
iv. 7wt% to 23wt % of 6SL and 3SL combined, preferably 8wt % to 22wt %; and
v. 10wt% to 40wt% of 3FL, preferably 11wt% to 37wt%

9. The composition according to any one of the preceding claims which is a nutritional composition selected from the list consisting of an infant formula, a starter infant formula, a follow-on or follow-up formula, a baby food, an infant cereal composition, growing-up-milk, a fortifier such as a human milk fortifier, or a supplement.

10. A composition as defined in any one of the preceding claims for use in: i) preventing and/or treating bacterial infections in an infant or a young child; ii) modulating the microbiota of an infant or a young child in need thereof; and/or iii) preventing and/or treating allergy of an infant or a young child.

11. A composition for use according to claim 10, wherein the use is for modulating the microbiota of an infant or a young child in need thereof and administration of said composition results in abundance of Bifidobacteriaceae and/or *Bifidobacterium longum* subsp. *infantis* being increased.

12. The composition for use according to claim 10, wherein the use is for modulating the microbiota of an infant or a young child in need thereof; and/or preventing and/or treating allergy of an infant or a young child by increasing intestinal short-chain fatty acids (SCFA) production in such infant or young child.

## Patentansprüche

1. Zusammensetzung für Säuglinge oder Kleinkinder, umfassend mindestens einen probiotischen Bakterienstamm, wobei es sich bei dem probiotischen Bakterienstamm um Bifidobakterien handelt, und eine präbiotische Oligosaccharidmischung, bestehend aus 2'-Fucosyllactose (2FL), Lactodifucotetraose/Difucosyllactose (DFL), Lacto-N-tetraose (LNT), 6'-Sialyllactose (6SL) und 3'-Sialyllactose (3SL) und optional 3-Fucosyllactose (3FL);
wobei die Bifidobakterien *Bifidobacterium longum* Subsp. *infantis* oder eine Kombination aus *Bifidobacterium animalis* Subsp. *lactis* und *Bifidobacterium longum* Subsp. *infantis* umfassen;
wobei mindestens eines der Bifidobakterien *Bifidobacterium longum* Subsp. *infantis* LMG 11588 oder ein Stamm ist, der eine durchschnittliche Nukleotididentität (ANI) von mindestens 99,9 % mit diesem Stamm aufweist.

2. Zusammensetzung nach Anspruch 1, wobei die Bifidobakterien *Bifidobacterium longum* Subsp. *infantis* umfassen und die präbiotische Oligosaccharidmischung aus 2'-Fucosyllactose (2FL), Lactodifucotetraose/Difucosyllactose (DFL), Lacto-N-tetraose (LNT), 6'-Sialyllactose (6SL) und 3'-Sialyllactose (3SL) besteht.

3. Zusammensetzung nach Anspruch 1, wobei die Bifidobakterien eine Kombination aus *Bifidobacterium animalis* Subsp. *lactis* und *Bifidobacterium longum* Subsp. *infantis* ist und die präbiotische Oligosaccharidmischung aus 2'-Fucosyllactose (2FL), Lactodifucotetraose/Difucosyllactose (DFL), Lacto-N-tetraose (LNT), 6'-Sialyllactose (6SL) und 3'-Sialyllactose (3SL) besteht.

4. Zusammensetzung nach einem der Ansprüche 1 bis 3, wobei mindestens eines der Bifidobakterien *Bifidobacterium animalis* Subsp. *lactis* CNCM 1-3446 ist.

5. Zusammensetzung nach einem der vorstehenden Ansprüche, wobei die Mischung aus HMOs im Wesentlichen besteht aus:
i. 34 Gew.-% bis 85 Gew.-%, vorzugsweise 42 Gew.-% bis 71 Gew.-%, 2FL;
ii. 10 Gew.-% bis 40 Gew.-%, vorzugsweise 14 Gew.-% bis 26 Gew.-%, LNT;
iii. 4 Gew.-% bis 14 Gew.-%, vorzugsweise 5 Gew.-% bis 10 Gew.-%, DFL; und
iv. 9 Gew.-% bis 31 Gew.-%, vorzugsweise 10 Gew.-% bis 28 Gew.-%, 6SL und 3SL zusammen.

6. Zusammensetzung nach einem der Ansprüche 1 bis 4, wobei die Mischung aus HMOs im Wesentlichen besteht aus:
i. 26 Gew.-% bis 65 Gew.-%, vorzugsweise 32 Gew.-% bis 54 Gew.-%, 2FL;
ii. 8 Gew.-% bis 30 Gew.-%, vorzugsweise 11 Gew.-% bis 20 Gew.-%, LNT;
iii. 3 Gew.-% bis 11 Gew.-%, vorzugsweise 4 Gew.-% bis 8 Gew.-%, DFL;
iv. 7 Gew.-% bis 23 Gew.-%, vorzugsweise 8 Gew.-% bis 22 Gew.-%, 6SL und 3SL zusammen; und
v. 12 Gew.-% bis 38 Gew.-%, vorzugsweise 17 Gew.-% bis 31 Gew.-%, 3FL

7. Zusammensetzung nach einem der Ansprüche 1 bis 4, wobei die Mischung aus HMOs im Wesentlichen besteht aus:
i. 40 Gew.-% bis 80 Gew.-%, vorzugsweise 50 Gew.-% bis 70 Gew.-%, 2FL;
ii. 10 Gew.-% bis 40 Gew.-%, vorzugsweise 14 Gew.-% bis 26 Gew.-%, LNT;
iii. 4 Gew.-% bis 14 Gew.-%, vorzugsweise 5 Gew.-% bis 10 Gew.-%, DFL; und
iv. 9 Gew.-% bis 31 Gew.-%, vorzugsweise 10 Gew.-% bis 28 Gew.-%, 6SL und 3SL zusammen.

8. Zusammensetzung nach einem der Ansprüche 1 bis 4, wobei die Mischung aus HMOs im Wesentlichen besteht aus:
i. 20 Gew.-% bis 60 Gew.-%, vorzugsweise 25 Gew.-% bis 55 Gew.-%, 2FL;
ii. 8 Gew.-% bis 30 Gew.-%, vorzugsweise 11 Gew.-% bis 20 Gew.-%, LNT;
iii. 2 Gew.-% bis 12 Gew.-%, vorzugsweise 4 Gew.-% bis 8 Gew.-%, DFL;
iv. 7 Gew.-% bis 23 Gew.-%, vorzugsweise 8 Gew.-% bis 22 Gew.-%, 6SL und 3SL zusammen; und
v. 10 Gew.-% bis 40 Gew.-%, vorzugsweise 11 Gew.-% bis 37 Gew.-%, 3FL

9. Zusammensetzung nach einem der vorstehenden Ansprüche, wobei es sich um eine Nahrungsmittelzusammensetzung handelt, die aus der Liste ausgewählt ist, bestehend aus einer Säuglingsanfangsnahrung, einer Starter-Säuglingsanfangsnahrung, einer Folge- oder Nachfolgenahrung, einer Babynahrung, einer Getreidezusammensetzung für Säuglinge, einer Wachstumsmilch, einem Stärkungsmittel wie ein Stärkungsmittel für menschliche Milch oder einem Ergänzungsmittel.

10. Zusammensetzung nach einem der vorstehenden Ansprüche zur Verwendung bei: i) einem Vorbeugen und/oder Behandeln bakterieller Infektionen bei einem Säugling oder einem Kleinkind; ii) einem Modulieren der Mikroflora eines Säuglings oder eines Kleinkinds, das dies benötigt;
und/oder iii) Vorbeugen und/oder Behandeln einer Allergie bei einem Säugling oder einem Kleinkind.

11. Zusammensetzung zur Verwendung nach Anspruch 10, wobei die Verwendung zum Modulieren der Mikroflora eines Säuglings oder eines Kleinkinds dient, das dies benötigt,
und die Verabreichung der Zusammensetzung dazu führt, dass eine Menge von Bifidobacteriaceae und/oder *Bifidobacterium longum* Subsp. *infantis* erhöht wird.

12. Zusammensetzung zur Verwendung nach Anspruch 10, wobei die Verwendung zum Modulieren der Mikroflora eines Säuglings oder eines Kleinkinds, das dies benötigt; und/oder zum Vorbeugen und/oder Behandeln einer Allergie eines Säuglings oder eines Kleinkinds durch Erhöhen einer Produktion kurzkettiger Fettsäuren (SCFA) in dem Darm eines derartigen Säuglings oder Kleinkinds dient.

## Revendications

1. Composition pour nourrissons ou jeunes enfants comprenant au moins une souche bactérienne probiotique, ladite souche bactérienne probiotique étant des bifidobactéries, et un mélange d'oligosaccharides prébiotiques constitué de 2'-fucosyllactose (2FL), lactodifucotétraose/difucosyllactose (DFL), lacto-N-tétraose (LNT), 6'-sialyllactose (6SL), 3'-sialyllactose (3SL) et éventuellement 3-fucosyllactose (3FL) ;
dans laquelle les bifidobactéries comprennent *Bifidobacterium longum* sous-espèce *infantis* ou une combinaison de *Bifidobacterium animalis* sous-espèce *lactis* et *Bifidobacterium longum* sous-espèce *infantis ;*
dans laquelle au moins une des bifidobactéries est LMG 11588 *Bifidobacterium longum* sous-espèce *infantis* ou une souche ayant une identité nucléotidique moyenne (ANI) d'au moins 99,9 % avec cette souche.

2. Composition selon la revendication 1, dans laquelle les bifidobactéries comprennent *Bifidobacterium longum* sous-espèce *infantis* et le mélange d'oligosaccharides prébiotiques est constitué de 2'-fucosyllactose (2FL), lactodifucotétraose/difucosyllactose (DFL), lacto-N-tétraose (LNT), 6'-sialyllactose (6SL) et 3'-sialyllactose (3SL).

3. Composition selon la revendication 1, dans laquelle les bifidobactéries sont une combinaison de *Bifidobacterium animalis* sous-espèce *lactis* et *Bifidobacterium longum* sous-espèce *infantis* et le mélange d'oligosaccharides prébiotiques est constitué de 2'-fucosyllactose (2FL), lactodifucotétraose/difucosyllactose (DFL), lacto-N-tétraose (LNT), 6'-sialyllactose (6SL) et 3'-sialyllactose (3SL).

4. Composition selon l'une quelconque des revendications 1 à 3, dans laquelle au moins une des bifidobactéries est CNCM 1-3446 *Bifidobacterium animalis* sous-espèce *lactis.*

5. Composition selon l'une quelconque des revendications précédentes, dans laquelle le mélange d'HMO est essentiellement constitué de :
i. 34 % en poids à 85 % en poids de 2FL, de préférence de 42 % en poids à 71 % en poids ;
ii. 10 % en poids à 40 % en poids de LNT, de préférence de 14 % en poids à 26 % en poids ;
iii. 4 % en poids à 14 % en poids de DFL, de préférence de 5 % en poids à 10 % en poids ; et
iv. 9 % en poids à 31 % en poids de 6SL et 3SL combinés, de préférence de 10 % en poids à 28 % en poids.

6. Composition selon l'une quelconque des revendications 1 à 4, dans laquelle le mélange de HMO est constitué essentiellement de :
i. 26 % en poids à 65 % en poids de 2FL, de préférence de 32 % en poids à 54 % en poids ;
ii. 8 % en poids à 30 % en poids de LNT, de préférence de 11 % en poids à 20 % en poids ;
iii. 3 % en poids à 11 % en poids de DFL, de préférence de 4 % en poids à 8 % en poids ;
iv. 7 % en poids à 23 % en poids de 6SL et 3SL combinés, de préférence de 8 % en poids à 22 % en poids ; et
v. de 12 % en poids à 38 % en poids de 3FL, de préférence de 17 % en poids à 31 % en poids

7. Composition selon l'une quelconque des revendications 1 à 4, dans laquelle le mélange de HMO est constitué essentiellement de :
i. 40 % en poids à 80 % en poids de 2FL, de préférence de 50 % en poids à 70 % en poids ;
ii. 10 % en poids à 40 % en poids de LNT, de préférence de 14 % en poids à 26 % en poids ;
iii. 4 % en poids à 14 % en poids de DFL, de préférence de 5 % en poids à 10 % en poids ; et
iv. 9 % en poids à 31 % en poids de 6SL et 3SL combinés, de préférence de 10 % en poids à 28 % en poids.

8. Composition selon l'une quelconque des revendications 1 à 4, dans laquelle le mélange de HMO est constitué essentiellement de :
i. 20 % en poids à 60 % en poids de 2FL, de préférence de 25 % en poids à 55 % en poids ;
ii. 8 % en poids à 30 % en poids de LNT, de préférence de 11 % en poids à 20 % en poids ;
iii. 2 % en poids à 12 % en poids de DFL, de préférence de 4 % en poids à 8 % en poids ;
iv. 7 % en poids à 23 % en poids de 6SL et 3SL combinés, de préférence de 8 % en poids à 22 % en poids ; et
v. 10 % en poids à 40 % en poids de 3FL, de préférence de 11 % en poids à 37 % en poids

9. Composition selon l'une quelconque des revendications précédentes, qui est une composition nutritionnelle choisie parmi la liste constituée d'une préparation pour nourrissons, une préparation de première étape pour nourrissons, une préparation de suite ou de suivi, une alimentation pour bébés, une composition de céréale pour nourrissons, un lait de croissance, un fortifiant tel qu'un fortifiant pour lait humain, ou un complément.

10. Composition selon l'une quelconque des revendications précédentes, pour utilisation dans : i) la prévention et/ou le traitement des infections bactériennes chez un nourrisson ou un jeune enfant ; ii) la modulation du microbiote d'un nourrisson ou d'un jeune enfant qui a besoin de celui-ci ;
et/ou iii) la prévention et/ou le traitement de l'allergie d'un nourrisson ou d'un jeune enfant.

11. Composition pour utilisation selon la revendication 10, dans laquelle l'utilisation consiste à moduler le microbiote d'un nourrisson ou d'un jeune enfant qui a besoin de celui-ci
et l'administration de ladite composition entraîne une augmentation de l'abondance des Bifidobacteriaceae et/ou de *Bifidobacterium longum* sous-espèce *infantis.*

12. Composition pour utilisation selon la revendication 10, dans laquelle l'utilisation consiste à moduler le microbiote d'un nourrisson ou d'un jeune enfant qui a besoin de celui-ci ;
et/ou la prévention et/ou le traitement de l'allergie d'un nourrisson ou d'un jeune enfant en augmentant la production intestinale d'acides gras à chaîne courte (AGCC) chez ce nourrisson ou ce jeune enfant.
